# EUROPEAN PATENT APPLICATION

(11) **EP 3 730 112 A1**
(43) Date of publication of application: **28.10.2020**
(21) Application number: 19201174.0
(22) Date of filing: 02.10.2019
(51) Int. Cl.: A61F 13/537, A61F 13/49, D04H 3/013, D04H 3/14

(54) **ACQUISITION/DISTRIBUTION LAYER AND ABSORBENT HYGIENE ARTICLE CONTAINING SAID ACQUISITION/DISTRIBUTION LAYER**

(71) Applicant: LENZING AKTIENGESELLSCHAFT, 4860 Lenzing (AT)
(72) Inventor: CARLYLE, Tom, Holly Springs, NC 27540 (US); GOLDHALM, Gisela, 3363 Neufurth (AT); MAYER, Katharina, 4813 Altmünster (AT)

(57) **Abstract**

The invention relates to an acquisition/distribution layer (ADL) for use in an absorbent hygiene article, comprising at least one nonwoven layer. It is in object of the invention to provide a biodegradable ADL with improved dimensional stability and liquid absorbency properties. To solve the stated problem, it is therefore proposed, that the at least one nonwoven layer is a cellulosic nonwoven layer, directly manufactured from lyocell spinning solution, forming a network of substantially endless regenerated cellulosic fibers.

## Description

### Technical Field

The present invention relates to an acquisition/distribution layer (ADL) for use in an absorbent hygiene article, comprising at least one nonwoven layer, and an adsorbent hygiene article containing said acquisition/distribution layer.

### Background Art

ADLs are regularly used in a variety of adsorbent hygiene articles, such as baby diapers, feminine hygiene sheets, adult incontinence diapers, pads and underpants, etc. ADLs for these products share a number of common requirements for a reliable functionality. They require good liquid absorbency and liquid wicking properties and high dimensional stability, even in the wet state. Furthermore fast liquid take-up/acquisition and good spread-ability of liquids (in order to distribute the liquid uniformly over the surface of the product rather than only near the liquid introduction point) are key parameters. Additionally, biodegradability and sustainability become an ever more important criterion for disposable hygiene articles.

Current nonwoven ADLs, however, fail to fulfill all of the above mentioned needs. Synthetic fiber based carded and spunlace nonwovens and products incorporating them, on the one hand, are not very absorbent or biodegradable or sustainably sourced. Cellulosic nonwovens or products incorporating cellulosic fibers, on the other hand, typically are not dimensionally stable, especially when wet, and may not be strong enough for use in ADLs.

There are several types of nonwovens known for use in ADL applications. Airlaid and coform nonwovens are known and used in hygiene products. US 5,879,343 and US 5,938,995 teach the manufacture of airlaid nonwovens, while US 5,843,063, US 8,598,406 and US 7,923,597 all describe the manufacture of coform or coform-type nonwovens (meltblown/airlaid composites or spunbond/airlaid composites).

ADL made of airlaid and coform nonwovens are absorbent and hydrophilic, but wicking, liquid spread-ability and wet strength are issues, as is linting. Additionally, they use at least partly non-biodegradable raw materials.

Carded and hydroentangled or spunlace nonwovens are used in hygiene ADL applications. US 9,642,754, US 9,750,651, US 10,206,826 and WO 2017/095399 all teach the use of carded nonwovens, either resin bonded or through air bonded, in hygiene products. Many of these are 100% synthetic fiber based.

ADL made of hydroentangled or spunlace nonwovens and carded nonwovens are often compromise products, using either 100% or high percentages of hydrophobic, synthetic fibers to minimize rewet and improve liquid spread-ability. In exchange, they have marginal absorbency, slow strike through and are usually not 100% biodegradable.

Thus, there is distinct need for a strong, soft, absorbent, liquid wicking, fast liquid uptaking, good liquid spreading, comfortable, dimensionally stable (even wet), low linting, cost effective, biodegradable and sustainable nonwoven material for use in ADL applications.

### Disclosure of the Invention

Thus it is an object of the present invention to provide an ADL of the aforementioned type, which shows improved dimensional stability and liquid absorbency properties, while at the same time being biodegradable.

The present invention achieves the stated object in that the at least one nonwoven layer is a cellulosic nonwoven layer, directly manufactured from lyocell spinning solution, forming a network of substantially endless regenerated cellulosic fibers.

If the nonwoven fabric is a cellulosic nonwoven fabric, consisting of substantially endless regenerated cellulosic fibers, which form a network of substantially endless regenerated cellulosic fibers, the nonwoven layer being directly manufactured from lyocell spinning solution, an easy to produce yet reliable ADL with high dimensional stability may be provided. The cellulosic fibers can further improve the liquid wicking and liquid absorbency properties of the ADL.

Furthermore, the inventive ADL may contain essentially only cellulose, thus, showing good biodegradability. Such an essentially pure cellulose nonwoven layer may be formed directly from lyocell spinning solution and is preferably multibonded by merged filaments, hydrogen bonding and/or physical intermingling of the filaments to form the network of essentially continuous regenerated cellulosic fibers. Thus, the dimensional stability of the ADL may be further improved.

The cellulosic nonwoven layer, directly manufactured from lyocell spinning solution, constituting the ADL of the present invention is manufactured according to a novel variant of a spunlaid nonwoven process, e.g. described in WO 2018/184038 and WO 2018/184932. In this process, a (lyocell) cellulose spinning mass is extruded simultaneously through a spinneret containing closely-spaced meltblown jet nozzles. The extruded spinning solution is then attenuated using high velocity air streams and the nonwoven web/layer is formed on a moving surface. Finally the nonwoven web is washed and dried. By carefully adjusting the spinning parameters (cellulose concentration of the spinning mass, spinneret extrusion rate, attenuating air stream velocity, etc.), the strength- and liquid-transport-properties of the formed nonwoven can be tailored to the needs.

Such inventive ADL formed by the above mentioned process may thus provide good absorbency, fast wicking, fast liquid uptake, good spread-ability of liquid, enhanced dimensional stability (wet and dry), biodegradability and sustainability; all without the need of adding binders or chemicals.

It is mentioned in general, that the ADL containing essentially only cellulose takes into account, that regenerated molded bodies, like fibers or filaments spun according to the lyocell process, may contain residual amounts of other substances than cellulose, e.g. hemicelluloses. This does, however, not affect the quality of the product or its ability for use according to this invention in any way.

The at least one nonwoven layer of the ADL may be additionally treated by hydroentangling, hydroembossing and/or perforating to further improve the dimensional stability of the ADL.

Further advantageous embodiments of the invention are shown in accordance with the dependent claims 2 to 11.

The present invention further relates to an absorbent hygiene article, comprising an absorbent core and an ADL according to any of claims 1 to 11 arranged over the surface of the absorbent core, in order to homogeneously distribute liquid over the surface of the absorbent core.

### Modes for Carrying Out the Invention

In the following, the invention is described in further detail in accordance to a number of preferred embodiments. All described embodiments may be combined with each other without restrictions, if not stated otherwise.

In a first embodiment, the inventive acquisition/distribution layer (ADL) comprises a first nonwoven layer, that is directly manufactured from lyocell spinning solution. The first nonwoven layer contains substantially endless regenerated cellulosic fibers, which form a network of substantially endless regenerated cellulosic fibers. A ADL with fast liquid uptake, good spread-ability of liquid, good liquid wicking, good absorbency and good dimensional stability (wet and dry) can be made. All of this is achieved with the ADL being 100% biodegradable, compostable and made from 100% sustainable raw materials.

Additionally, the regenerated cellulosic fibers in the first nonwoven layer are multibonded by merging, hydrogen bonding and/or physically intermingling in order to form the network of essentially endless regenerated cellulosic fibers, resulting in a ADL with high dimensional stability. This multibonding may result directly from the manufacturing process or through additional steps. The advantages of the multibonding used in the inventive ADL is that low elongation and good stiffness are achieved. This combination enables the material to exhibit very good dimensional stability as an essentially pure cellulosic material. It also provides the low linting character that comes from good filament tie-down (merged filaments) and does not negatively influence the excellent softness that cellulosic materials are known to exhibit, and this translates into a comfortable, biodegradable material.

In another embodiment, the ADL of the nonwoven material according to the invention is additionally bonded or treated by a hydroentanglement, hydroembossing, perforation, needlepunch or chemical bonding process to modify the physical properties. Such modified properties may be for example bulk density, tensile strength and elongation.

In a further embodiment, the inventive ADL has a dry elongation of less than or equal to 20 % in MD (machine direction) and less than or equal to 50 % in CD (cross direction). In a yet further embodiment, the ADL has a dry elongation of less than or equal to 10 % in MD, preferably less than or equal to 7 % in MD. In another further embodiment, the ADL has a dry elongation of less than or equal to 30 % in CD, preferably less than or equal to 20 % in CD.

In a further embodiment, the inventive ADL has a wet elongation of less than or equal to 25 % in MD and less than or equal to 50 % in CD. In a yet further embodiment, the ADL has a wet elongation of less than or equal to 20 % in MD, preferably less than or equal to 15 % in MD. In another further embodiment, the ADL has a wet elongation of less than or equal to 40 % in CD, preferably less than or equal to 30 % in CD.

The methods for measuring the dry and wet elongation are described in the examples section. All elongation values in this disclosure - if not stated otherwise explicitly - refer to measurements taken in line with this methods.

In another embodiment, the inventive ADL has a liquid absorbency of higher than 3 g/g, i.e. 3 grams of liquid (water) per gram of the ADL.

The method for measuring the liquid absorbency used throughout this disclosure is described in detail in the examples section.

In a preferred embodiment, the basis weight of the ADL is between 20 and 120 g/m² (gsm - grams per square meter). In a further preferred embodiment, the basis weight of the ADL is between 35 and 120 g/m². Within this range, we find that inventive ADL product performances can be advantageously achieved.

In a further preferred embodiment, the ADL comprises multiple cellulosic nonwoven layers of substantially endless regenerated cellulosic fibers which are directly formed on top of each other in one single process step and which are interconnected by means of merging and/or hydroentanglement to form the network of substantially endless regenerated cellulosic fibers. All layers are directly manufactured from lyocell spinning solution on top of each other in one process step.

In yet a further embodiment, the different cellulosic nonwoven layers, directly formed on top of each other, have different wicking properties. The wicking properties can be adjusted by adjusting the unique production process, such that the integrally formed network of substantially endless regenerated cellulosic fibers has a gradient in liquid wicking properties throughout the nonwoven layers formed on top of each other.

In yet a further embodiment, the ADL comprises at least 2 cellulosic nonwoven layers which are directly formed from lyocell spinning solution on top of each other in one process step, which differ in their properties. The upper layer(s) provide the properties for use as a liquid permeable topsheet in hygiene articles, with higher softness, having finer fibers and high liquid strike through, while the layer(s) beneath have an increased liquid spread-ability, having higher diameter fibers and higher merging of fibers. This all will be produced in a single production step by making adjustments in the production process, as for example with modification of spinning air pressure, coagulation flow or coagulation droplet size. The resulting inventive ADL can be used both as an ADL and a topsheet in an absorbent hygiene article at the same time.

In another embodiment, the nonwoven layers in the ADL are combined with another nonwoven material to produce a nonwoven composite that forms the ADL. The nonwoven layers are integrally formed composite nonwoven products in themselves, and they may be combined with individual components that are composites as well. In one embodiment, the nonwoven layers are combined with chemically cross-linked cellulose ("curly") fibers. Such a nonwoven composite material would provide the inventive strength, dimensional stability and wicking while the chemically cross-linked cellulose fibers would provide lower rewet, and faster strike-through properties.

In a further embodiment, the nonwoven layers in the ADL are combined with other nonwoven materials through hydroentanglement, hydroembossing and/or perforating. Thereby, the ADL comprises an additional layer formed on top of the network of substantially endless fibers, whereby the additional layer consists of cellulosic pulp or staple fibers, more particularly lyocell fibers, and the additional layer is hydroentangled, hydroembossed or perforated with the network of substantially endless fibers.

In order to modify wicking, or other liquid handling properties of the nonwoven material for use in ADL products according to the invention it can be treated with chemicals, polymers or other materials.

In another embodiment, the nonwoven layers within the ADL are treated with chemicals, polymers or other materials to modify rewet, hydrophobicity or other liquid handling properties. The individual nonwoven layers or the network of substantially endless cellulosic fibers as a whole can be treated accordingly.

The invention also discloses an absorbent hygiene article comprising an absorbent core and an ADL according to any number of embodiments above, arranged over the surface of the absorbent core, in order to homogeneously distribute liquid over the surface of said absorbent core.

In another embodiment, the absorbent hygiene article further comprises a backsheet. The absorbent core is then arranged between the ADL and said backsheet. The ADL should thereby acquire and pass the liquid quickly to the absorbent core and distribute the liquid uniformly over the whole surface of the absorbent core, while the liquid impermeable backsheet should hold the liquid inside the absorbent core.

In another embodiment, the ADL additionally acts as the topsheet for the absorbent hygiene article, thus ADL and topsheet are integrally formed as different cellulosic nonwoven layers. In yet another embodiment, the ADL additionally acts as both the topsheet and the absorbent core for the absorbent hygiene article, thus the topsheet, the ADL and the absorbent core are integrally formed in one production process as different cellulosic nonwoven layers with different properties on top of each other.

In further embodiments, such an absorbent hygiene article is a baby diaper, a feminine care pad, an adult incontinence product or a tampon. Nevertheless, many other absorbent hygiene articles, where the properties of the inventive ADL are beneficial, are in the scope of this invention.

### Examples

In the following, the invention will be illustrated by examples. These examples are not limiting the scope of the invention in any way. The invention also includes any other embodiments which are based on the same inventive concept.

All samples for testing were conditioned at 23°C ±2°C rel. humidity 50% ±5% for 24 hours.

### Example 1:

A 35 g/m² product of the inventive ADL was tested for nonwovens demand absorbency versus a comparative commercial ADL product of the same basis weight, comprising air bonded bico PE/PET and PP. Demand absorbency was measured using EDANA test method NWSP 010.3.R0 (15).

As test liquid, demineralised water was used.

Test WSP 10.3 describes the determination of the nonwoven absorbency when one side of a fabric is brought in contact with an aqueous liquid while the whole sample is kept under a constant mechanical pressure. In hygiene applications, high absorbency under slight mechanical pressure is a very important requirement. Within the spectrum of EDANA and INDA test methods the demand absorbency test WSP 10.3 is the closest approximation to simulate these end-uses.

The commercial product had a demand absorbency of only 0.47 g/g (grams of water per grams of ADL); while the inventive ADL averaged to 6.6 g/g. This represents an improvement of approximately 1300% over the commercial product.

### Example 2:

The same two samples of example 1 were further tested for spread-ability of liquid.

The test method was as follows: 0.5 ml of test liquid (water with 2 g/L dye - Sulfacide brilliant green) was pipetted onto each sample using an Eppendorf pipette. After 1 day, a picture of the liquid spread was taken and software (ImageJ1.49v, National Institute of Health, USA) used to evaluate the area of the liquid spread.

The inventive ADL showed a 12 fold higher area in spread liquid compared to the commercial product.

### Example 3:

Further samples of the ADL according to the invention (inventive examples E1 to E3) and commercial ADL-products (comparative examples C1 to C3) were tested for dimensional stability, in particular elongation in machine direction (MD) and cross direction (CD). The inventive ADL shows significant better dimensional stability.

The results are summarized in Table 1.

**Table 1: Dry and wet elongation in MD and CD measured for 3 different inventive examples (E1 to E3) and 3 comparative examples (C1 to C3).**

| sample | dry elongation MD [%] | dry elongation CD [%] | wet elongation MD [%] | wet elongation CD [%] |
|---|---|---|---|---|
| E1 (inventive example 1) | 1,7 | 5,0 | 7,4 | 23,6 |
| E2 (inventive example 2) | 2,1 | 5,4 | 8,0 | 19,2 |
| E3 (inventive example 3) | 5,2 | 8,5 | 14,9 | 28,4 |
| C1 (comparative example 1) | 31,5 | 70,0 | 33,3 | 77,7 |
| C2 (comparative example 2) | 22,1 | 51,6 | 28,5 | 70,5 |
| C3 (comparative example 3) | 30,1 | 73,2 | 32,1 | 88,2 |

Prior to all measurements, the samples were first conditioned at 23 °C (± 2 °C) and 50 % (± 5 %) relative humidity for 24 h. For all measurements, the samples had a size of 5 cm × 10 cm (width × length). The clamping length was 8 cm and the speed for testing 10 cm/min.

Elongations in MD and CD (in dry and wet state) were measured according to DIN EN 29 073 part 3 / ISO 9073-3 (in the version of year 1992).

To determine dry elongation, the samples were measured after the conditioning described above.

To determine wet elongation, the samples were wetted out 3 fold with demineralized water and were sealed within a plastic bag for 60 min to equilibrate moisture.

## Claims

1. An acquisition/distribution layer (ADL) for use in an absorbent hygiene article, comprising at least one nonwoven layer, **characterized in that**, the at least one nonwoven layer is a cellulosic nonwoven layer, directly manufactured from lyocell spinning solution, forming a network of substantially endless regenerated cellulosic fibers.

2. The ADL according to claim 1, **characterized in that** the ADL has a liquid absorbency of higher than 3 g/g.

3. The ADL according to claim 1 or 2, **characterized in that** the ADL has a dry elongation of less than or equal to 20 % in MD, more particularly of less than or equal to 10 % in MD, preferably of less than or equal to 7 % in MD, and of less than or equal to 50 % in CD, more particularly of less than or equal to 30 % in CD, preferably of less than or equal to 20 % in CD.

4. The ADL according to any of claims 1 to 3, **characterized in that** the ADL has a wet elongation of less than or equal to 25 % in MD, more particularly of less than or equal to 20 % in MD, preferably of less than or equal to 15 % in MD, and of less than or equal to 50 % in CD, more particularly of less than or equal to 40 % in CD, preferably of less than or equal to 30 % in CD.

5. The ADL according to any of claims 1 to 4, **characterized in that** the at least one cellulosic nonwoven layer exhibits modified physical properties due to additional hydroentanglement, hydroembossing, perforating, needlepunching and/or chemical bonding processes.

6. The ADL according to any of claims 1 to 5, **characterized in that** the substantially endless regenerated cellulosic fibers in the cellulosic nonwoven layer are multibonded by merging, hydrogen bonding and/or physical intermingling of said fibers.

7. The ADL according to any of claims 1 to 6, **characterized in that** the ADL comprises multiple cellulosic nonwoven layers of substantially endless regenerated cellulosic fibers which are directly formed on top of each other in one single process step and which are interconnected by means of merging and/or hydroentanglement to form the network of substantially endless regenerated cellulosic fibers.

8. The ADL according to claim 7, **characterized in that** the different cellulosic nonwoven layers have different wicking properties.

9. The ADL according to any of claims 1 to 8, **characterized in that** the ADL comprises an additional layer formed on top of the network of substantially endless fibers, whereby the additional layer consists of cellulosic pulp or staple fibers, more particularly lyocell fibers, and the additional layer is hydroentangled, hydroembossed or perforated with the network of substantially endless fibers.

10. The ADL according to any of claims 1 to 9, **characterized in that** the ADL has a basis weight between 20 and 120 g/m².

11. The ADL according to any of claims 1 to 10, **characterized in that** the ADL has a modified absorbency, wicking, or other liquid handling property due to a treatment with chemicals or polymers.

12. An absorbent hygiene article comprising an absorbent core and an acquisition distribution layer (ADL) according to any of claims 1 to 11 arranged over the surface of the absorbent core, in order to homogeneously distribute liquid over the surface of the absorbent core.

13. The absorbent hygiene article according to claim 12, **characterized in that** it further comprises a backsheet, whereby the absorbent core is arranged between the ADL and said backsheet.

14. The absorbent hygiene article according to any of claims 12 or 13, **characterized in that** the ADL additionally acts as the topsheet for the absorbent hygiene article.

15. The absorbent hygiene article according to any of claims 12 to 14, **characterized in that** the absorbent hygiene article is a baby diaper, feminine care pad, adult incontinence pad or tampon.
